# EUROPÄISCHE PATENTANMELDUNG

(11) **EP 3 266 475 A1**
(43) Veröffentlichungstag der Anmeldung: **10.01.2018**
(21) Anmeldenummer: 16178371.7
(22) Anmeldetag: 07.07.2016
(51) Int. Cl.: A61M 1/10, A61M 1/12

(54) **BLUTPUMPE ZUR HERZUNTERSTÜTZUNG**

(71) Anmelder: Berlin Heart GmbH, 12247 Berlin (DE)
(72) Erfinder: Nüsser, Peter, 14532 Kleinmachnow (DE); Müller, Jörg, 10439 Berlin (DE); Wisniewski, Adrian, 10963 Berlin (DE); Wurm, Frank-Hendrik, 44265 Dortmund (DE); Hallier, Sebastian, 18055 Rostock (DE)
(74) Vertreter: Pfenning, Meinig & Partner mbB

(57) **Zusammenfassung**

Die Erfindung bezieht sich auf eine Blutpumpe zur Herzunterstützung mit einem Rotor (4), der innerhalb eines Rotorraumes (6a) rotiert und der Förderelemente (10) aufweist, die das Blut in Umfangsrichtung und/oder in Radialrichtung fördern, wobei anschließend an den Rotorraum ein Auslassgehäuse vorgesehen ist. Die Neuerung sieht eine im Auslassgehäuse angeordnete Strömungsleitvorrichtung in Form wenigstens einer oder mehrerer Schaufeln (20, 21, 22, 31, 36, 40) vor, deren Längserstreckung entlang einer Begrenzungswand des Auslassgehäuses verläuft. Mittels der Schaufeln kann die Blutströmung in Bezug auf die Effizienz, Effektivität der Pumpe und die Blutschädigung optimiert werden.

## Beschreibung

Die Erfindung liegt auf dem Gebiet der Mechanik und der Strömungsmechanik und ist mit besonderem Vorteil im medizinischen Bereich anwendbar.

Konkret bezieht sich die Erfindung auf eine Blutpumpe zur Herzunterstützung und ihre optimierte Ausgestaltung.

Blutpumpen zur Herzunterstützung sind seit einiger Zeit in vielfältigen Ausprägungen bekannt und werden üblicherweise an einer Herzkammer angeschlossen, um von dort Blut zu einem anderen Anschluss im Blutkreislauf eines Patienten zu fördern. Hierdurch wird vorübergehend oder dauerhaft eine Herzfunktion oder die Funktion einer Herzkammer unterstützt oder ersetzt.

Viele derartige Pumpen weisen antreibbare Rotoren auf, die mittels schaufelartiger Förderelemente Blut von einem Pumpeneinlass zu einem Pumpenauslass fördern. Eine übliche Aufgabe besteht dabei darin, die Pumpe möglichst effizient zu gestalten, um Antriebsenergie zu sparen, dabei die Baugröße möglichst klein zu halten und eine möglichst große Förderleistung zu verwirklichen. Um bei geringer Baugröße eine zufriedenstellende Förderleistung zu erreichen, ist oft eine hohe Drehzahl des Pumpenrotors notwendig. Eine weitere Randbedingung hierbei ist, dass durch den Pumpvorgang die Blutbestandteile des zu fördernden Blutes in möglichst geringem Maß geschädigt werden sollen.

Eine Pumpe der eingangs genannten Art ist beispielsweise aus der Druckschrift WO 2011/054545 A1 bekannt. Dort ist eine Pumpe mit einem Rotor beschrieben, der ein schneckenförmig umlaufendes Förderelement aufweist und Blut entlang der Längsachse des Rotors in Axial- und Radialrichtung fördert.

Der vorliegenden Neuerung liegt vor dem Hintergrund des Standes der Technik die Aufgabe zugrunde, bei einer Blutpumpe der eingangs genannten Art eine möglichst gute Förderleistung mit einer geringen Beschädigung zu verbinden.

Die Aufgabe wird gemäß der Neuerung mit den Merkmalen des Patentanspruchs 1 gelöst. Die Patentansprüche 2 bis 12 stellen Möglichkeiten zur Realisierung der beanspruchten Lehre dar. Die Patentansprüche 13 bis 15 beziehen sich auf ein Verfahren zum Herstellen einer derartigen Blutpumpe.

Demgemäß bezieht sich die Erfindung auf eine Blutpumpe zur Herzunterstützung mit einem Rotor, der innerhalb eines Rotorraumes rotiert und der Förderelemente aufweist, die das Blut in Umfangsrichtung und/oder in Radialrichtung fördern, wobei anschließend an den Rotorraum ein Auslassgehäuse vorgesehen ist. Dabei ist zur Realisierung der Neuerung eine im Auslassgehäuse angeordnete Strömungsleitvorrichtung vorgesehen, die wenigstens ein Strömungsleitelement in Form einer Schaufel aufweist, deren Längserstreckung entlang einer Begrenzungswand des Auslassgehäuses verläuft.

Durch derartige Strömungsleitelemente in Form von Schaufeln soll im Zusammenwirken mit dem rotierenden Rotor eine möglichst günstige Steuerung der Blutströmung erreicht werden. Ein Aspekt dabei ist, dass durch die Funktion eines Pumpenrotors das Blut nicht nur in der gewünschten axialen oder radialen Richtung gefördert wird, sondern zudem einen Drehimpuls durch seine Bewegung in Umfangsrichtung des Rotors erhält. Diese Bewegung des Blutes in Umfangsrichtung des Rotors kann beispielsweise mittels der Strömungsleitvorrichtung(en) wenigstens teilweise in eine radiale und/oder axiale Bewegungsrichtung in Bezug auf den Rotor umgelenkt werden. Dies verbessert den Wirkungsgrad der Pumpe. Dies führt dazu, dass die Drehzahl der Pumpe bei gleicher Leistung gegenüber bekannten Pumpen reduziert und damit die Blutschädigung reduziert werden kann.

Eine oder mehrere Strömungsleitelemente in Form von Schaufeln weisen dazu jeweils eine Längserstreckung auf, die üblicherweise den Verlauf der Schaufel entlang ihrer längsten Längenausdehnung markiert. Senkrecht zu dieser Längserstreckungsrichtung werden die Breite und die Höhe der Schaufel gemessen. Die Höhe der Schaufel ist dabei üblicherweise größer als die Breite. Die Höhe erstreckt sich üblicherweise von der Begrenzungswand des Auslassgehäuses, an der die Schaufel befestigt ist, senkrecht weg.

Das bedeutet, dass eine oder mehrere Schaufeln im Wesentlichen mit ihrer Längserstreckung entlang einer Begrenzungswand des Auslassgehäuses angeordnet sind. Im einfachsten Fall, auf den die vorliegende Neuerung jedoch nicht beschränkt ist, kann eine solche Schaufel als ein an einer Begrenzungswand verlaufender, nicht notwendigerweise gerader und in seiner Längserstreckungsrichtung gegenüber der Breite und Höhe langer Steg ausgebildet sein. Die Höhe des Steges kann beispielsweise größer sein als die Breite.

Eine besondere Ausgestaltung der Neuerung kann vorsehen, dass die Schaufel(n) wenigstens abschnittsweise mit der Begrenzungswand des Auslassgehäuses verbunden ist/sind und mit einer Höhe, die geringer ist als ihre Länge, entlang der Längserstreckung in den Innenraum des Auslassgehäuses hinein-ragt/-ragen. In diesem Fall kann eine Seite der Schaufel(n) ganz oder teilweise mit einer Begrenzungswand des Auslassgehäuses verbunden sein, wobei die dieser Seite gegenüberliegende Seite als Oberkante der Schaufel frei in das Innere des Auslassgehäuses ragt.

Derartige Schaufeln können beispielsweise an einer Stirnseite eines Auslassgehäuses vorgesehen sein, das sich in Verlängerung der Axialrichtung des Pumpenrotors befindet. Das durch den Rotor geförderte Blut kann dann axial auf die Stirnwand des Auslassgehäuses prallen und gleichzeitig einen Drehimpuls mitbringen, d. h. eine Strömungsrichtung in Umfangsrichtung des Rotors. Trifft die Strömung dann auf die Stirnseite des Auslassgehäuses, so wird diese Strömungsrichtung des Blutes in eine Radialrichtung umgesetzt, die üblicherweise radial nach außen führt, so dass das geförderte Blut radial außen am Auslassgehäuse zu einem Pumpenauslass gefördert wird, wo es radial/tangential aus dem Auslassgehäuse entweicht.

Es kann zudem vorgesehen sein, dass die wenigstens eine Schaufel sich von einer ersten Wand des Auslassgehäuses bis zu einer zweiten Wand des Auslassgehäuses erstreckt, die der ersten Wand gegenüberliegt. In diesem Fall kann eine Schaufel innerhalb des Auslassgehäuses eine Unterteilung des Innenraums des Auslassgehäuses schaffen und auf diese Weise die Blutströmung beeinflussen. Es kann somit vorgesehen sein, dass wenigstens eine Schaufel eine Teilungswand des Auslassgehäuses bildet.

Es kann zudem vorgesehen sein, dass eine Schaufel an einer stirnseitigen Wand des Auslassgehäuses wenigstens teilweise in Umfangsrichtung des Rotors verläuft, insbesondere spiralförmig in Strömungsrichtung des geförderten Blutes von radial innen nach außen. Eine oder mehrere Schaufeln können auf diese Weise durch ihre Anordnung in Spiralform, die in der Rotationsrichtung des Rotors gesehen von radial innen nach radial außen verläuft, die Blutströmung von einer stärker in Umfangsrichtung gerichteten Ausrichtung in eine stärker radial gerichtete Ausrichtung umlenken. Hierzu können eine oder mehrere Schaufeln spiralförmig ausgebildet sein, beispielsweise auch jeweils einen Abschnitt einer einzigen durchgehenden, gedachten Spirale darstellen. Die Schaufeln können jedoch auch gerade verlaufen und radial von innen nach außen, wobei sie vorteilhaft nicht auf die Rotorachse ausgerichtet sind, sondern tangential zu einem um die Rotorachse konzentrisch verlaufenden Kreis.

Eine weitere Ausgestaltung der Neuerung kann vorsehen, dass wenigstens eine Schaufel sich im Querschnitt von ihrer Basis, die mit der Wand des Auslassgehäuses verbunden ist, zum Innenraum des Auslassgehäuses hin verjüngt, insbesondere spitz zuläuft. Eine solche Schaufel kann somit als Steg ausgebildet sein, der entlang der Längserstreckung der Schaufel eine gleichbleibende oder veränderliche Höhe aufweist. Erweist im Querschnitt eine Dreiecksform auf und ähnelt in der Form damit der Form einer Fischflosse.

Es kann auch vorgesehen sein, dass wenigstens eine Seitenwand einer Schaufel im Querschnitt konkav geformt ist. Eine solche Ausformung der Seitenwände der Schaufeln verbessert die Effizienz der Umlenkung der Blutströmung weiter.

Außerdem kann vorgesehen sein, dass die Höhe der wenigstens einen Schaufel sich entlang ihrer Längserstreckung in Bewegungsrichtung des geförderten Blutes verringert oder vergrößert. Beispielsweise kann in einem mittleren Bereich die Schaufel ihre höchste Erhebung von der Wand des Auslassgehäuses aus erreichen und zu beiden Enden der Längserstreckung in der Höhe abnehmen.

Es kann auch vorgesehen sein, dass eine Schaufel vom ersten Ende ihrer Längserstreckung bis zum zweiten Ende der Längserstreckung kontinuierlich bezüglich der Höhe zu- oder abnimmt.

Eine weitere Ausgestaltung der Neuerung kann vorsehen, dass die Schaufel(n) wenigstens teilweise aus einem elastisch nachgiebigen Werkstoff besteht/ bestehen. Damit kann bei sich verändernden Strömungsbedingungen und einem höheren Fluiddruck die jeweilige Schaufel durch flexibles, elastisches Nachgeben an die Blutströmung angepasst werden, um für verschiedene Strömungsbedingungen einen optimierten Betrieb bzw. eine optimierte Effizienz zu gewährleisten. Damit kann auch sichergestellt werden, dass unter Extrembedingungen die Blutschädigung in Grenzen gehalten wird.

Als nachgiebiges Material der Schaufel kann ein Elastomer, Gummi oder ein in gewissen Grenzen elastischer Kunststoff vorgesehen sein.

Eine weitere Ausgestaltung der Neuerung kann vorsehen, dass mehrere Schaufeln vorgesehen sind, von denen jede in ihrer Längserstreckung einen Kreisbogenabschnitt oder den Abschnitt einer Spirale bildet, und wobei die Schaufeln in Umfangsrichtung des Rotors hintereinander angeordnet sind. Durch eine derartige Anordnung mehrerer Schaufeln kann insgesamt eine optimierte Blutströmung im Rahmen der Strömungslenkung realisiert werden. Dabei können beispielsweise an einer Stirnwand des Auslassgehäuses drei Schaufeln um die Rotationsachse gleichmäßig verteilt sein.

Es kann gemäß der Neuerung zudem vorgesehen sein, dass das Auslassgehäuse die Form einer Einfachspirale oder Doppelspirale oder die Form eines Schneckengehäuses aufweist. Für bestimmte Einsatzfälle kann die Gehäuseform einer Doppelspirale für das Auslassgehäuse Vorteile mit sich bringen.

Eine weitere Ausgestaltung der Neuerung kann vorsehen, dass die Schaufeln im Auslassgehäuse derart angeordnet und geformt sind, das der Drehimpuls des geförderten Blutes gegenüber einer Ausgestaltung ohne diese Schaufeln verringert ist, insbesondere derart, dass die Strömung des Blutes von einer ersten, in Axialrichtung und in Umfangsrichtung des Rotors verlaufenden Strömungsrichtung in eine zweite, in Umfangsrichtung und stärker in Radialrichtung des Rotors verlaufende Strömungsrichtung umgelenkt wird.

Ein vorteilhaftes Verfahren zur Herstellung einer Pumpe der oben beschriebenen Art kann vorsehen, dass die Schaufeln mit dem Auslassgehäuse gemeinsam in einem Gießverfahren mit verlorener Form hergestellt werden. Da das Auslassgehäuse besonders bezüglich seines Innenraums mitsamt der in diesem integrierten Schaufeln eine sehr komplexe geometrische Form darstellt und aus einer Gießform schwer zu entformen ist, bietet sich eine Herstellung in verlorener Form im Gießverfahren an.

Es kann jedoch auch vorgesehen sein, dass bei der Herstellung des Auslassgehäuses Befestigungsvorrichtungen, insbesondere Ausnehmungen mit Hinterschneidungen oder Stege, vorgesehen werden, an denen Schaufeln befestigbar sind. In diesem Fall ist die Herstellung des eigentlichen Auslassgehäuses vereinfacht, jedoch können die Schaufeln der Strömungsleitvorrichtung nach der Herstellung des Auslassgehäuses einzeln eingesetzt und befestigt werden.

Alternativ können die Schaufeln auch durch Fräsen und/oder durch Erodieren hergestellt werden. Dies geschieht beispielsweise durch zumindest bereichsweises Fräsen und/oder bereichsweises Erodieren eines Auslassgehäuserohkörpers.

Im Folgenden wird die Neuerung anhand von Figuren einer Zeichnung in Ausführungsbeispielen gezeigt und nachfolgend erläutert. Dabei zeigt
- Fig. 1: im Längsschnitt eine Blutpumpe mit einem Rotor,
- Fig. 2: den Rotor einer Blutpumpe mit einer Förderschaufel, wobei das Gehäuse der Pumpe weggelassen und ein Auslassgehäuse sichtbar ist,
- Fig. 3: einen ersten Schnitt durch ein Auslassgehäuse,
- Fig. 4: einen Schnitt durch ein weiteres Auslassgehäuse,
- Fig. 5: einen Schnitt durch ein weiteres Auslassgehäuse,
- Fig. 6: einen weiteren Schnitt durch ein Auslassgehäuse mit einem Doppelspiralraum,
- Fig. 7: eine Seitenansicht einer Schaufel,
- Fig. 8: die Seitenansicht einer weiteren Schaufel,
- Fig. 9: eine weitere Seitenansicht einer Schaufel,
- Fig. 10: einen ersten, zweiten und dritten Querschnitt verschiedener Schaufeln sowie
- Fig. 11: eine vierten, fünften und sechsten Querschnitt verschiedener Schaufeln.

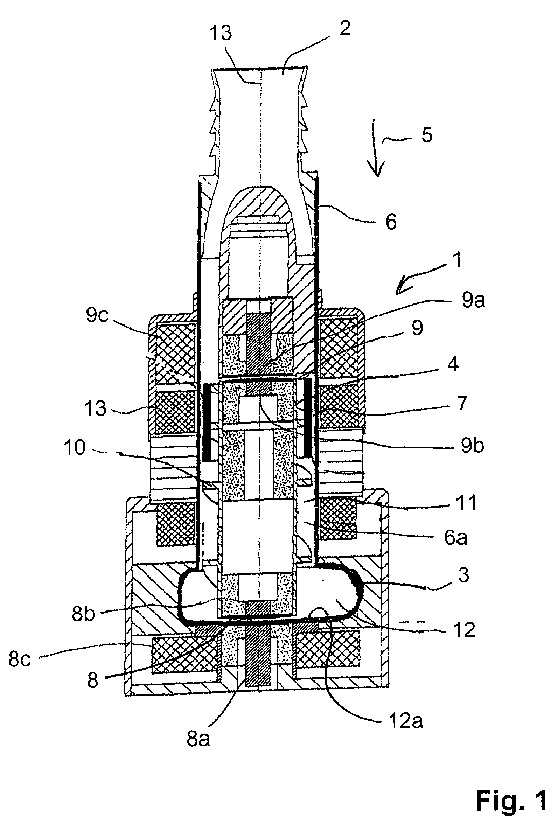

In Figur 1 ist schematisch in einem Längsschnitt die Blutpumpe 1 dargestellt, die einen Pumpeneinlass 2 und einen Pumpenauslass 3 aufweist, wobei durch Förderelemente eines Rotors 4 in Axialrichtung 5 Blut gefördert wird. Das Pumpengehäuse 6 der Pumpe 1 weist einen zylindrischen Hohlraum (Rotorraum 6a) auf, in dem der Rotor 4 rotiert. Der Rotor 4 ist dabei mittels eines radialen hydrodynamischen Lagers 7 an der Innenwand des Gehäuses 6 abgestützt. Zwei magnetische Axiallager 8, 9 weisen jeweils einen feststehenden Magneten 8a, 9a, einen im Rotor mitrotierenden bewegten Magneten 8b, 9b sowie jeweils eine strombeaufschlagte Spule 8c, 9c zur geregelten Erzeugung eines Magnetfeldes auf. Ein nicht im Einzelnen näher dargestellter Positionssensor ermittelt eine Axialposition des Rotors 4, die zur Regelung dieser Axialposition in eine Stromstärke der Steuerspule 8c, 9c umgesetzt wird. Der Rotor 4 ist auf diese Weise sehr reibungsarm und zuverlässig auch für hohe Drehzahlen gelagert.

Am Umfang des Rotors 4 ist eine schneckenartig umlaufende Schaufel 10 angeordnet, die durch den Ringspalt 11 das Blut bis zum Auslassgehäuse 12 fördert. Die axiale Komponente der Blutströmung bewirkt, dass das Blut zunächst vor die stirnseitige Wand 12a prallt. Das Blut weist dort eine Strömungskomponente in Umfangsrichtung des Rotors 4 zusätzlich zur Axialkomponente auf. An der Stirnseite 12a des Auslassgehäuses 12 sind Strömungsleitelemente in Form von Schaufeln fest angeordnet und mit der Wand 12a verbunden. Auf die Form und Ausrichtung der Strömungsleitelemente wird weiter unten noch näher eingegangen.

Der Antrieb des Rotors 4 geschieht mittels einer Elektromotoranordnung, bei der Rotormagnete im Rotor 4 selbst angeordnet sind, während der Stator mit Statorwicklungen 13 im Pumpengehäuse 2 angeordnet ist. Das Auslassgehäuse 12 ist als spiralförmiges Gehäuse um die Achse 13 der Pumpe herum angeordnet. Die Blutströmung, die im Auslassgehäuse in eine radiale Strömungskomponente und eine Strömungskomponente in Umfangsrichtung um die Achse 13 umgelenkt wird, kann tangential aus dem Pumpenauslass herausströmen.

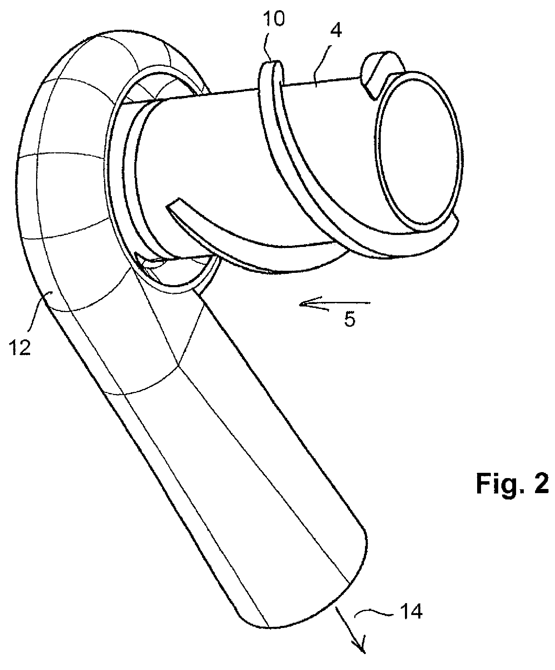

In Figur 2 ist in perspektivischer Ansicht der Rotor 4 mit der Förderschaufel 10 dargestellt. Das Blut wird entlang der Achse des Rotors in der Richtung 5 zum Auslassgehäuse 12 gefördert. Die spiralartige Ausgestaltung des Auslassgehäuses 12 ist in Figur 2 gut zu erkennen. Das Blut strömt in Richtung des Pfeils 14 aus dem Auslassgehäuse 12 heraus.

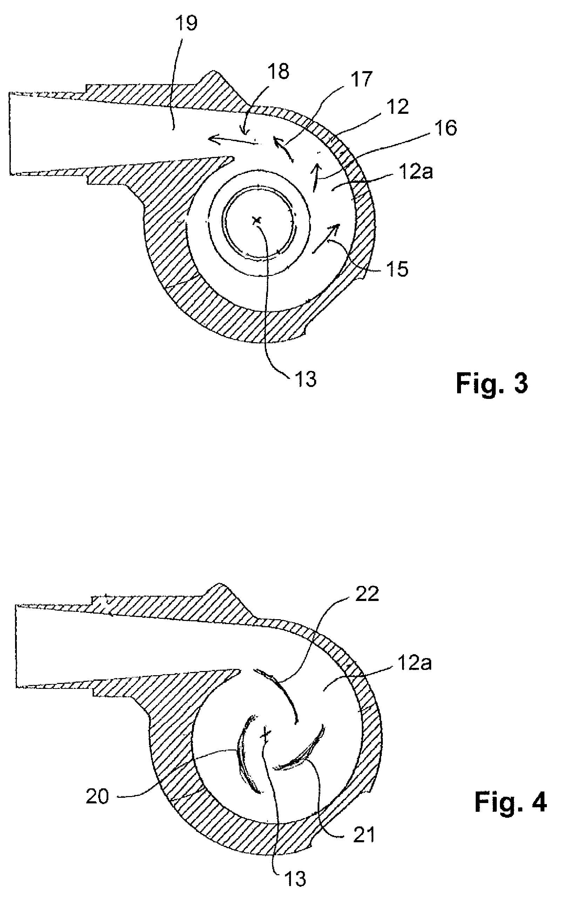

Figur 3 zeigt einen Querschnitt durch ein Auslassgehäuse 12 senkrecht zur Achse 13 in Figur 1, d. h. senkrecht zur Rotorachse. Die Blutströmung prallt auf die stirnseitige Wand 12a des Auslassgehäuses 12 und wird insgesamt in eine Strömung in Umfangsrichtung und radial nach außen bezüglich der Rotorachse 13 umgelenkt, wie durch die Pfeile 15, 16, 17 angedeutet ist. Die Blutströmung verlässt das Auslassgehäuse 12 in tangentialer Richtung bezüglich des Rotors, angedeutet durch den Pfeil 18, zum Pumpenauslass 19.

In Figur 4 sind an der stirnseitigen Gehäusewand 12a des Auslassgehäuses 12 drei Strömungsleitelemente in Form von Schaufeln 20, 21, 22 dargestellt. Die drei Schaufeln 20, 21, 22 verlaufen in ihrer Längserstreckung jeweils parallel zur stirnseitigen Wand 12a des Auslassgehäuses in der oder parallel zur Zeichenebene und sind in ihrer Längserstreckung leicht spiralförmig oder kreisbogenförmig gekrümmt. Die einzelnen Schaufeln sind jeweils als Spiralabschnitte ausgebildet, wobei jede einzelne Schaufel Teil einer gemeinsamen, von der Rotorachse 13 ausgehenden Spirale sein kann. Jede einzelne der Schaufeln ist als ein Steg ausgebildet, dessen Länge in Längserstreckung gegenüber der Höhe, mit der die jeweilige Schaufel in den Auslassraum 12 hineinragt, und gegenüber der Breite des jeweiligen Stegs groß ist. Die Höhe der Schaufeln 20, 21, 22 kann jedoch auch so hoch sein, dass die Schaufeln den gesamten Innenraum des Auslassgehäuses 12 durchsetzen und damit abschnittsweise geschlossene Kanäle im Auslassgehäuse 12 bilden.

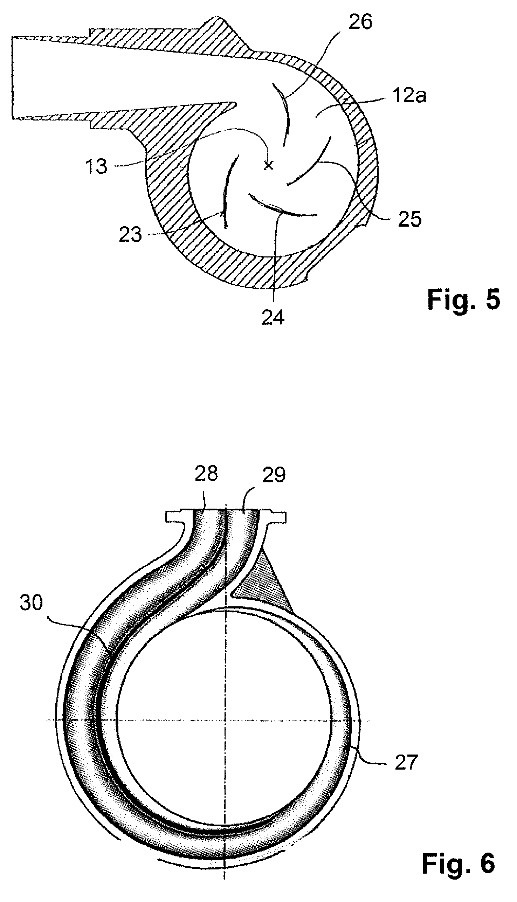

In Figur 5 sind in einem Querschnitt eines Auslassgehäuses, der den Ansichten aus Figur 3 und Figur 4 entspricht, auf der Wand 12a eines Auslassgehäuses vier Schaufeln 23, 24, 25, 26 gezeigt, die in Umfangsrichtung bezüglich der Rotorachse 13 hintereinanderliegen und gegeneinander um jeweils etwa 90° um die Rotorachse 13 versetzt sind. Es ist auch eine höhere Anzahl von Schaufeln, beispielsweise fünf oder zwischen fünf und zehn Schaufeln, an der Stirnwand 12a denkbar, wobei dann zwischen benachbarten Schaufeln geringere Winkelabstände vorliegen.

Figur 6 zeigt beispielhaft ein Auslassgehäuses 27, das zwei parallel zueinander verlaufende Spiralen 28, 29 aufweist, die durch eine Trennwand 30 voneinander getrennt sind. Auch eine solche Gestaltung des Auslassgehäuses kann geeignete Strömungsverhältnisse der Blutströmung erzeugen.

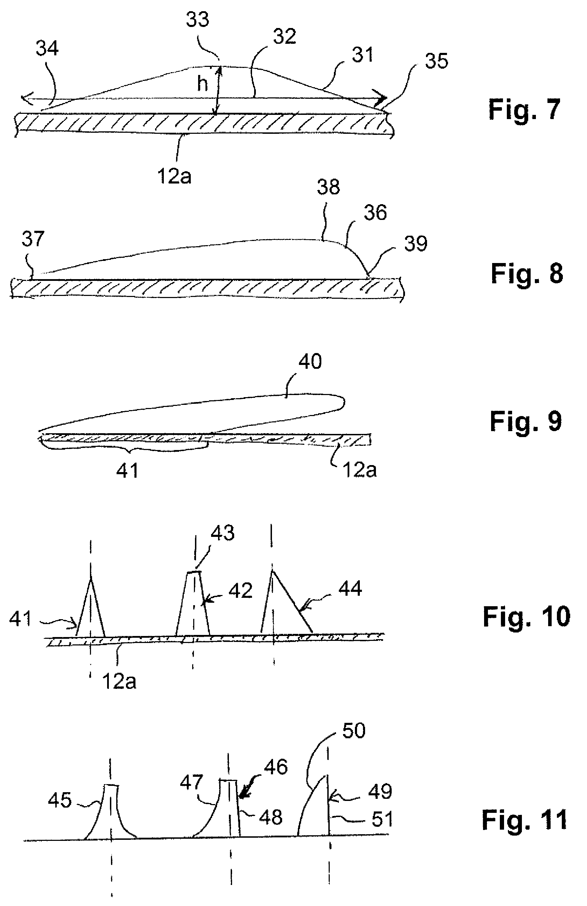

In Figur 7 ist eine Schaufel in einer Queransicht gezeigt, wobei die Längserstreckung der Schaufel 31 mit dem Pfeil 32 angedeutet ist. Die Schaufel 31 kann dabei senkrecht zur Zeichenebene auch gekrümmt verlaufen. Die Richtung der Längserstreckung 32 folgt dann dem gekrümmten Verlauf.

Unterhalb der Schaufel 31 ist die Stirnwand 12a des Auslassgehäuses dargestellt. Die in Figur 7 dargestellte Schaufel 31 weist ihre größte Höhe h im Mittenbereich am Punkt 33 auf, und ihre Höhe verringert sich zu beiden Enden 34, 35 stetig auf null.

Bei der in Figur 8 in einer Seitenansicht gezeigten Schaufel 36 steigt die Höhe vom ersten Ende 37 bis zum höchsten Punkt 38 langsam an, um dann zum zweiten Ende 39 hin steil abzufallen. Die stegartigen Schaufeln 31, 36 sind jeweils entlang ihrer gesamten Längserstreckung mit der Wand 12a des Auslassgehäuses verbunden, beispielsweise einstückig durch gemeinsames Gießen des Auslassgehäuses mit den Schaufeln.

In Figur 9 ist eine Schaufel 40 gezeigt, die nur in einem Bereich 41 ihrer Länge mit der Wand 12a des Auslassgehäuses verbunden ist und über den Bereich 41 hinaus frei in das Innere des Auslassgehäuses hineinragt.

In Figur 10 sind drei Beispiele für mögliche Querschnitte der Schaufeln/Stege dargestellt, wobei bei dem am weitesten links liegenden Beispiel 41 ein symmetrischer dreieckiger, von der Wand 12a aus spitz zulaufender Querschnitt gezeigt ist, im Beispiel 42 ein sich von der Wand 12a weg verjüngender, in einer Abflachung 43 endender Querschnitt und bei dem Beispiel 44 ein asymmetrisch dreieckiger Querschnitt.

In Figur 11 sind drei mögliche Querschnittsformen für Schaufeln angegeben, die jeweils gekrümmte Flanken aufweisen, wobei in dem Beispiel 45 zwei konkave Flanken einer Schaufel symmetrisch vorgesehen sind, beim Beispiel 46 eine konkave Flanke 47 und eine gerade Flanke 48 und beim Beispiel 49 eine konvexe Flanke 50 sowie eine gerade Flanke 51.

Die diskutierten Parameter Länge der Schaufeln in Längserstreckungsrichtung, Höhe, Höhenverlauf, Breite sowie Profilform und Anzahl und Anordnung an einer Stirnwand eines Auslassgehäuses oder auch in anderen Bereichen des Pumpengehäuses können derart ausgestaltet und kombiniert werden, dass über einen großen Dynamikbereich der Förderungsleistung optimierte Förderbedingungen zur Förderung von Blut erreicht werden. Es ist zu betonen, dass die vorliegende Neuerung für verschiedene Rotorarten und Arten der Strömungsführung sowie für verschiedene Formen von Auslassgehäusen, wie beispielsweise einfache oder doppelte Spiralform oder eine sich in zwei Dimensionen erweiternde Schneckengehäuseform, anwendbar ist. Durch geeignete Gestaltung der genannten Parameter lassen sich über einen großen Drehzahlbereich der Pumpe akzeptable Werte in Bezug auf die Blutschädigung, die Querkräfte auf den Rotor sowie den Wirkungsgrad erzielen. Dadurch kann eine geringe Blutschädigung bei einem gleichzeitig durch die Pumpe erreichbaren hohen Druckgefälle erreicht werden.

## Patentansprüche

1. Blutpumpe zur Herzunterstützung mit einem Rotor (4), der innerhalb eines Rotorraumes (6a) rotiert und der Förderelemente aufweist, die das Blut in Umfangsrichtung und/oder in Radialrichtung fördern, wobei anschließend an den Rotorraum 6a ein Auslassgehäuse (12) vorgesehen ist, **gekennzeichnet durch** eine im Auslassgehäuse angeordnete Strömungsleitvorrichtung, die wenigstens ein Strömungsleitelement in Form einer Schaufel (20, 21, 22, 31, 36, 40) aufweist, deren Längserstreckung entlang einer Begrenzungswand (12a) des Auslassgehäuses verläuft.

2. Blutpumpe nach Anspruch 1, **dadurch gekennzeichnet, dass** die Schaufel (20, 21, 22, 31, 36, 40) wenigstens abschnittsweise mit der Begrenzungswand (12a) des Auslassgehäuses (12) verbunden ist und mit einer Höhe, die geringer ist als seine Länge entlang der Längserstreckung, in den Innenraum des Auslassgehäuses (12) hineinragt.

3. Blutpumpe nach Anspruch 1, **dadurch gekennzeichnet, dass** die wenigstens eine Schaufel (20, 21, 22, 31, 36,40) sich von einer ersten Wand (12a) des Auslassgehäuses (12) bis zu einer zweiten Wand des Auslassgehäuses erstreckt, die der ersten Wand gegenüberliegt.

4. Blutpumpe nach Anspruch 1 oder 3, **dadurch gekennzeichnet, dass** wenigstens eine Schaufel (20, 21, 22, 31, 36, 40) eine Teilungswand des Auslassgehäuses bildet.

5. Blutpumpe nach Anspruch 1 oder einem der folgenden, **dadurch gekennzeichnet, dass** eine Schaufel (20, 21, 22, 31, 36, 40) an einer stirnseitigen Wand des Auslassgehäuses (12) wenigstens teilweise in Umfangsrichtung des Rotors (4) verläuft, insbesondere spiralförmig in Strömungsrichtung des geförderten Blutes von radial innen nach außen.

6. Blutpumpe nach Anspruch 1 oder einem der folgenden, **dadurch gekennzeichnet, dass** die Schaufel (20, 21, 22, 31, 36, 40) sich im Querschnitt von ihrer Basis, die mit der Wand (12a) des Auslassgehäuses (12) verbunden ist, zum Innenraum des Auslassgehäuses hin verjüngt, insbesondere spitz zuläuft.

7. Blutpumpe nach Anspruch 1 oder einem der folgenden, **dadurch gekennzeichnet, dass** wenigstens eine Seitenwand (45,47) der Schaufel im Querschnitt konkav geformt ist.

8. Blutpumpe nach Anspruch 1 oder einem der folgenden, **dadurch gekennzeichnet, dass** die Höhe (h) der wenigstens einen Schaufel (31, 36, 40) sich entlang ihrer Längserstreckung in Bewegungsrichtung des geförderten Blutes verringert oder vergrößert.

9. Blutpumpe nach Anspruch 1 oder einem der folgenden, **dadurch gekennzeichnet, dass** die Schaufel(n) (20, 21, 22, 31, 36, 40) wenigstens teilweise aus einem elastisch nachgiebigen Werkstoff besteht/bestehen.

10. Blutpumpe nach Anspruch 1 oder einem der folgenden, **dadurch gekennzeichnet, dass** mehrere Schaufeln (20, 21, 22, 31, 36,40) vorgesehen sind, von denen jede in ihrer Längserstreckung einen Kreisbogenabschnitt oder den Abschnitt einer Spirale bildet, wobei die Schaufeln in Umfangsrichtung des Rotors (4) hintereinander angeordnet sind.

11. Blutpumpe nach einem der vorhergehenden Ansprüche, **dadurch gekennzeichnet, dass** das Auslassgehäuse (12) die Form einer Einfachspirale oder Doppelspirale oder die Form eines Schneckengehäuses aufweist.

12. Blutpumpe nach einem der vorhergehenden Ansprüche, **dadurch gekennzeichnet, dass** die Schaufeln (20, 21, 22, 31, 36,40) im Auslassgehäuse (12) derart angeordnet und geformt sind, dass der Drehimpuls des geförderten Blutes gegenüber einer Ausgestaltung ohne diese Schaufeln verringert ist, insbesondere derart, dass die Strömung des Blutes von einer ersten, in Axialrichtung und in Umfangsrichtung des Rotors (4) verlaufenden Strömungsrichtung in eine zweite, in Umfangsrichtung und in Radialrichtung des Rotors verlaufende Strömungsrichtung umgelenkt wird.

13. Verfahren zur Herstellung einer Blutpumpe nach einem der vorangehenden Ansprüche, **dadurch gekennzeichnet, dass** die Schaufeln (20, 21, 22, 31, 36,40) mit dem Auslassgehäuse (12) gemeinsam in einem Gießverfahren, und zwar vorzugsweise in einem Gießverfahren mit verlorener Form, hergestellt werden.

14. Verfahren zum Herstellen einer Blutpumpe nach einem der Ansprüche 1 bis 12, **dadurch gekennzeichnet, dass** bei der Herstellung des Auslassgehäuses Befestigungsvorrichtungen, insbesondere Ausnehmungen mit Hinterschneidungen oder Stege, vorgesehen werden, an denen Schaufeln (20, 21, 22, 31, 36,40) befestigbar sind.

15. Verfahren nach einem der Ansprüche 1 bis 12, **dadurch gekennzeichnet, dass** mindestens eine im Auslassgehäuse angeordnete Strömungsleitvorrichtung, die wenigstens ein Strömungsleitelement in Form einer Schaufel aufweist, deren Längserstreckung entlang einer Begrenzungswand des Anschlussgehäuses verläuft, durch Erodieren und/oder durch Fräsen eines Auslassgehäuserohkörpers hergestellt wird.
